# EUROPÄISCHE PATENTANMELDUNG

(11) **EP 1 759 682 A2**
(43) Veröffentlichungstag der Anmeldung: **07.03.2007**
(21) Anmeldenummer: 06118474.3
(22) Anmeldetag: 04.08.2006
(51) Int. Cl.: A61K 6/04, A61C 13/00

(54) **Verfahren zur Herstellung metallischer Bauteile, entsprechende metallische Bauteile sowie Kit zur Durchführung des Verfahrens**

(30) Priorität: 10.08.2005 DE 102005038074
(71) Anmelder: BEGO Bremer Goldschlägerei Wilh. Herbst GmbH & Co. KG, 28359 Bremen (DE)
(72) Erfinder: Laschütza, Helmut, 27721, Ritterhude (DE); Wiest, Thomas, 36088, Hünfeld (DE); Dierkes, Stephan, 28195, Bremen (DE); Eilers, Jan, 27283, Verden (DE)
(74) Vertreter: Eisenführ, Speiser & Partner

(57) **Zusammenfassung**

Beschrieben wird ein Verfahren zur Herstellung eines metallischen Bauteils, mit folgenden Schritten:
- Bereitstellen eines Negativmodells für das Bauteil, welches (i) zumindest an seiner Oberfläche elektrisch leitfähig ist oder leitfähig gemacht ist oder (ii) porös ist und im Innern eine Elektrode umfasst,
- Bereitstellen eines Schlickers der elektrophoretisch abscheidbare metallische und gegebenenfalls zusätzlich keramische Partikel umfasst,
- Elektrophoretisches Abscheiden einer Schicht der Partikel aus dem Schlicker auf der Oberfläche des Modells,
- Sintern oder Ansintern der abgeschiedenen Schicht der Partikel.

## Beschreibung

Die vorliegende Erfindung betrifft ein Verfahren zur Herstellung metallischer Bauteile, insbesondere einer Dentalrestauration auf Metallbasis, entsprechende metallische Bauteile (insbesondere Dentalrestaurationen) selbst sowie ein Kit zur Durchführung des erfindungsgemäßen Verfahrens.

Die Erfindung wird nachfolgend überwiegend mit Blick auf Dentalrestaurationen beschrieben, betrifft aber auch andere metallische Bauteile, vgl. insoweit auch die Beispiele 2 und 3.

Bei einer Dentalrestauration auf Metallbasis kann es sich insbesondere um einen metallischen Dentalkörper wie z.B. ein Käppchen, ein Inlay, Onlay oder eine Brücke handeln.

Gegebenfalls ist die Dentalrestauration aufbrennfähig, das heißt zum Aufbrennen einer keramischen Beschichtung vorgesehen.

Dentalrestaurationen auf Metallbasis, die als metallischer Zahnersatz eingesetzt werden sollen, werden vielfach über den Prozess des Gießens gewonnen. Bei diesem Prozess wird auf einem Meistermodell der zu restaurierenden Zahnsituation die Form des Zahnersatzes aus Wachs modelliert; das Meistermodell dient - bezogen auf die Wachsmodellation - als Negativmodell. Anschließend wird das Wachsmodell in eine Einbettmasse eingebettet. Aus dieser Einbettmasse wird das Wachs ausgebrannt und auf diese Weise eine Form gewonnen, die mit dem aufgeschmolzenen Metall ausgegossen wird. Die Verfahrensschritte werden im Dentallabor vor Ort durchgeführt, sind jedoch vergleichsweise arbeitsintensiv.

Weniger arbeitsintensiv für den Zahntechniker ist ein alternatives Verfahren, bei dem metallischer Zahnersatz (als Beispiel einer Dentalrestauration auf Metallbasis) in einer CAD/CAM-Kette erzeugt wird. Insoweit haben sich zwei alternative Wege in der Praxis etabliert. Gemäß beiden Wegen werden die über das Einscannen des Modells gewonnenen Daten mittels eines Rechners verarbeitet. In dem einen Fall wird durch Abtragen (Fräsen) eines metallischen Blockes die Dentalrestauration erzeugt. Im anderen Fall wird die Dentalrestauration lagenweise aufgebaut und einer lokalen Lasersinterung unterzogen. CAD/CAM-Ketten erfordern einen vergleichsweise hohen technischen Aufwand und den Einsatz vergleichsweise teurer Geräte. Regelmäßig werden die Dentalrestaurationen bei Einsatz von CAD/CAM-Ketten nicht im Dentallabor vor Ort hergestellt, sondern in speziellen Dentalzentren, in denen die erforderlichen CAD/CAM-Apparaturen vorhanden sind.

Es ist auch bereits bekannt, Goldgerüste galvanisch auf einem mittels Silberleitlack leitend gemachten Gipsstumpf abzuscheiden, der - bezogen auf das Goldgerüst - als Negativmodell dient. Im Verlaufe mehrerer Stunden wird beispielsweise eine etwa 0,2 bis 0,3mm dicke Goldschicht abgeschieden. Neben dem hohen Zeitbedarf wird es aber insoweit als nachteilig empfunden, dass eine Goldlösung Verwendung findet, deren Handhabung und Entsorgung nicht unproblematisch ist.

Es war daher die Aufgabe der vorliegenden Erfindung, ein Verfahren zur Herstellung eines metallischen Bauteils, insbesondere einer Dentalrestauration auf Metallbasis anzugeben, welches im Vergleich mit den klassischen Gießverfahren weniger arbeitsintensiv ist, im Vergleich mit den Verfahren unter Benutzung einer CAD/CAM-Kette einen geringeren technischen Aufwand erfordert und im Vergleich mit dem Verfahren der galvanischen Abscheidung eines Goldgerüsts schneller und hinsichtlich der Materialentsorgung weniger problematisch ist. Ferner sollte im Vergleich mit dem klassischen Gießverfahren und den CAD/CAM-Verfahren eine Modellation der Dentalrestauration (aus Wachs bzw. am Rechner) entfallen. Im Vergleich mit der galvanischen Abscheidung von Goldgerüsten sollte das anzugebende Verfahren auch den Einsatz anderer Metalle und von Legierungen ermöglichen.

Neben den vorstehend diskutierten Verfahren zur Herstellung von Dentalrestaurationen auf Metallbasis ist ein weiteres Verfahren bekannt, welches in der DE 35 32 331 A1 beschrieben ist. Gemäß dem dort beschriebenen Verfahren wird ein verblendbarer Zahnersatz mit metallischer Gefügematrix (das heißt eine Dentalrestauration auf Metallbasis) sintermetallurgisch hergestellt, indem mehrmodale Metallpulvermischungen, gegebenenfalls unter Zusatz von Glas- und Keramikpulvern, mit Wasser in einen Schlicker überführt werden, mit dem Schlicker der Zahnersatz modelliert wird und die Schlickermasse bei einer Temperatur gesintert wird, bei der die Solidustemperatur mindestens eines Pulverbestandteils überschritten wird. Das beschriebene Verfahren hat in der Praxis jedoch keine nennenswerte Bedeutung erlangt, insbesondere wohl deshalb, weil das manuelle Auftragen des Schlickers vergleichsweise aufwendig ist.

Weitere für das Verständnis der Bedeutung der vorliegenden Erfindung relevante Dokumente sind: DE 100 49 974 A1; DE 101 20 084 A1; DE 103 20 936 A1; US 5 238 751 A.

Die gestellte Aufgabe wird erfindungsgemäß gelöst durch ein Verfahren zur Herstellung eines metallischen Bauteils, insbesondere einer Dentalrestauration auf Metallbasis, mit den folgenden Schritten:
- Bereitstellen eines Negativmodells für das Bauteil, welches (i) zumindest an seiner Oberfläche elektrisch leitfähig ist oder leitfähig gemacht ist oder (ii) porös ist und im Innern eine Elektrode umfasst,
- Bereitstellen eines Schlickers, der elektrophoretisch abscheidbare metallische und gegebenenfalls zusätzlich keramische Partikel umfasst,
- Elektrophoretisches Abscheiden einer Schicht der Partikel aus dem Schlicker auf der Oberfläche des (Negativ)-Modells,
- Sintern oder Ansintern der abgeschiedenen Schicht der Partikel.

Insbesondere betrifft die Erfindung ein Verfahren zur Herstellung einer Dentalrestauration auf Metallbasis, mit folgenden Schritten:
- Bereitstellen eines Modells der zu restaurierenden Zahnsituation (bezogen auf die tatsächliche Zahnsituation ein Positiv, bezogen auf die Dentalrestauration aber ein Negativmodell), welches (i) zumindest an seiner Oberfläche elektrisch leitfähig ist oder leitfähig gemacht ist oder (ii) porös ist und im Innern eine Elektrode umfasst,
- Bereitstellen eines Schlickers, der elektrophoretisch abscheidbare metallische und gegebenenfalls zusätzlich keramische Partikel umfasst,
- Elektrophoretisches Abscheiden einer Schicht der Partikel aus dem Schlicker auf der Oberfläche des Modells,
- Sintern oder Ansintern der abgeschiedenen Schicht der Partikel.

Der Anteil metallischer Partikel in dem erfindungsgemäß eingesetzten Schlicker beträgt vorzugsweise 50-100 Gew.-%, bevorzugt 75-100 Gew.-%, bezogen auf die Gesamtmasse metallischer und keramischer Partikel. Vorzugsweise ist der Schlicker frei von keramischen Partikeln.

Erfindungsgemäß wird eine Schicht metallischer sowie gegebenenfalls (aber nicht notwendigerweise) keramischer Partikel elektrophoretisch auf der Oberfläche eines Negativmodells, vorzugsweise eines Modells einer zu restaurierenden Zahnsituation abgeschieden. Erforderlich zur Durchführung eines solchen Abscheidungsschrittes ist ein Negativmodell, z. B. ein Modell der zu restaurierenden Zahnsituation, bei dem (i) zumindest die Oberfläche elektrisch leitfähig ist oder leitfähig gemacht ist oder (ii) porös ist und im Inneren eine Elektrode umfasst.

Negativmodelle, z. B. Modelle einer zu restaurierenden Zahnsituation, die an ihrer Oberfläche elektrisch leitfähig sind, lassen sich aus leitfähigen Modellmaterialien herstellen, die z.B. erhältlich sind, indem üblichen, per se nicht leitfähigen Modellmaterialien wie Gips, feuerfesten Materialien, Wachs oder Kunststoff ein leitfähiges Material zugesetzt wird, beispielsweise Silber-, Graphit-, Ruß- oder Platin-Partikel. Alternativ ist die Verwendung von aus sich heraus leitfähigen Modellmaterialien möglich.

Ein Modell einer zu restaurierenden Zahnsituation, welches an seiner Oberfläche elektrisch leitfähig gemacht ist und, bezogen auf die fertige Dentalrestauration, als Negativmodell dienen kann, lässt sich beispielsweise durch Aufbringen einer leitfähigen Beschichtung auf ein Arbeitsmodell erhalten, welches per se nicht leitfähig ist. Als Beschichtungsmaterial können insbesondere Silberleitlack, Graphit- und Platinleitlack verwendet werden, alternativ können elektrisch leitfähige Schichten z.B. durch Aufsputtern von Metall appliziert werden. Dies gilt entsprechend für andere Negativmodelle.

Zur Erzeugung des Negativmodells (z. B. dentalen Arbeitsmodells) können die üblichen Verfahren eingesetzt werden, insbesondere die des Rapid Prototyping (Fräsen, Lasersintern, Ink-Jet-Printing, Stereolithographie, 3d-Printen) oder Abgussverfahren, insbesondere unter Verwendung von Dublierformen.

Zur Herstellung eines Modells der zu restaurierenden Zahnsituation, das porös ist und im Innern eine Elektrode umfasst, oder eines anderen Negativmodells ("negativ" bezogen auf das herzustellende metallische Bauteil) wird beispielsweise das Rapid Prototyping (Fräsen, 3d-Printen) oder Abgussverfahren, insbesondere unter Verwendung von Dublierformen verwendet. Die Aussparung innerhalb des Modells (oder des anderen Negativmodells), die zur Aufnahme der Elektrode benötigt wird, kann entweder direkt bei der Erzeugung des Modells eingebracht werden, oder es wird nachträglich z.B. durch Bohren eingebracht.

Ein Modell einer zu restaurierenden Zahnsituation (oder eine anderes Negativmodell), welches (i) zumindest an seiner Oberfläche elektrisch leitfähig ist oder leitfähig gemacht ist oder (ii) porös ist und im Inneren eine Elektrode umfasst, kann in einem elektrophoretischen Abscheidungsverfahren eingesetzt werden. Ziel ist dabei im Rahmen des erfindungsgemäßen Verfahrens die Abscheidung metallischer Partikel auf der Oberfläche des Modells, auch dann, wenn dieses porös ist und in seinem Inneren eine Elektrode umfasst; im letztgenannten Fall erfolgt die Abscheidung auf der Oberfläche des Modells nach Art einer Membranelektrophorese.

Vorzugsweise wird in einem erfindungsgemäßen Verfahren ein zumindest an seiner Oberfläche elektrisch leitfähiges oder leitfähig gemachtes Negativmodell, z. B.: ein Modell der zu restaurierenden Zahnsituation eingesetzt, welches umfasst:
- ein nicht nur an seiner Oberfläche elektrisch leitfähiges Modellmaterial ohne zusätzliche oberflächliche Beschichtung (also z.B. ein durch Zugabe von Silber-, Graphit-, Ruß- oder Platinpartikeln leitfähig gemachtes Material oder ein per se leitfähiges Material)
   oder
- ein elektrisch nicht leitendes Modell material mit einer oberflächlich darauf angeordneten Beschichtung aus einer leitfähigen Substanz (also insbesondere ein übliches nicht-leitfähiges Material wie Gips, feuerfestes Material, Wachs oder Kunststoff mit einer Beschichtung aus z.B. Silber, Graphit oder Platin).

Wird alternativ ein Negativmodell, insbesondere ein Modell einer zu restaurierenden Zahnsituation eingesetzt, das porös ist und im Inneren eine Elektrode umfasst, so ist die Porengröße des porösen Modells vorzugsweise so an die Partikelgröße der metallischen und (soweit vorhanden) zusätzlichen keramischen Partikel des Schlickers angepasst, dass die Partikel bei der Elektrophorese nicht bis zur Elektrode im Inneren des Modells vordringen können.

Derart an die Größe der Partikel angepasste Porengrößen sind für eine erfolgreiche Membranelektrophorese von hoher Wichtigkeit.

Gemäß einer ersten alternativen Verfahrensgestaltung besitzt das im erfindungsgemäßen Verfahren einzusetzende Negativmodell ("negativ" bezogen auf die fertige Dentalrestauration) die Abmessungen der zu restaurierenden Zahnsituation, ist also insbesondere gegenüber der zu restaurierenden Zahnsituation nicht vergrößert. Das Sintern der abgeschiedenen Schicht der Partikel (metallisch und eventuell zusätzlich keramisch) erfolgt dann vorzugsweise auf dem Modell, wobei das Modellmaterial so gewählt ist, dass das Modell unter den Sinterbedingungen eine relative Längenänderung von nicht mehr als 0,2% erfährt, vorzugsweise von nicht mehr als 0,05%, bezogen auf die Länge vor dem Sintern. Auch nach dem Sintern besitzt das Modell somit (zumindest nahezu) die Abmessungen der zu restaurierenden Zahnsituation. Die Außenabmessungen des Modells geben (zumindest nahezu) exakt die Innenabmessungen der gesinterten Schicht abgeschiedener metallischer Partikel vor. Auf Grund der Beibehaltung der Abmessungen des Modells unter den Sinterbedingungen, das heißt auf Grund der Abwesenheit eines nennenswerten Sinterschrumpfes für das Modell kann sich die Innengeometrie (die Innenabmessungen) der abgeschiedenen Schicht metallischer und eventuell keramischer Partikel während des Sinterprozesses nicht signifikant verändern. Es kommt aber zu einer Verringerung der Porosität innerhalb der Schicht metallischer Partikel und zu einer Reduzierung der Schichtdicke. Die Innengeometrie (die Innenabmessungen) einer beispielsweise so hergestellten Krone bleibt während des Sinterprozesses erhalten, so dass die Passgenauigkeit der Krone (oder einer anderen Dentalrestauration) besonders hoch ist. Das gesinterte, temperaturbeständige Modellmaterial, welches während des Sinterns keinem Sinterschrumpf unterlag, kann aus der gesinterten Schicht der metallischen Partikel mechanisch oder chemisch entfernt werden, z.B. durch Herausstrahlen oder Ätzen.

Auch gemäß einer zweiten alternativen Verfahrensgestaltung besitzt das im erfindungsgemäße Verfahren einzusetzende Modell die Abmessungen der zu restaurierenden Zahnsituation, ist also insbesondere gegenüber der zu restaurierenden Zahnsituation nicht vergrößert. Die Schicht der Partikel wird aber nur zur Festigkeitssteigerung angesintert (einer Wärmebehandlung unterzogen), d.h. es treten nur geringe relative Längenänderungen (kleiner 0,1% Längenänderung, vorzugsweise kleiner 0,05% Längenänderung, bezogen auf die Länge vor dem Ansintern) des Formkörpers auf. Die Porosität wird zudem nicht bzw. nicht wesentlich reduziert (vorzugsweise Änderung der Porosität um maximal 5%, bezogen auf die Porosität vor dem Ansintern). Das Modell wird in dieser Verfahrensgestaltung vorzugsweise so gewählt, dass es (i) bei einer Vorbehandlung (thermisch, mechanisch oder chemisch) vor dem Ansintern aus dem Formkörper (der Schicht metallischer Partikel) herausgelöst werden kann oder (ii) während des Ansinterns aus dem Formkörper herausschrumpft.

Bevorzugt ist ein erfindungsgemäßes Verfahren, in dem das Modell die Abmessungen der zu restaurierenden Zahnsituation besitzt, die abgeschiedene Schicht der beteiligten Partikel angesintert und gegebenenfalls zuvor einer Wärmebehandlung unterzogen wird, wobei beim Ansintern bzw. der vorausgehenden Wärmebehandlung das Modell material zusammenschrumpft bzw. aus der metallischen Schicht herausbrennt.

Gemäß einer dritten alternativen Verfahrensgestaltung besitzt das im erfindungsgemäßen Verfahren einzusetzende Modell größere Abmessungen als die zu restaurierende Zahnsituation. Das Sintern der abgeschiedenen Schicht der metallischen Partikel erfolgt dann auf dem Modell, wobei das Modellmaterial aber so gewählt ist, dass sich das Volumen des Modells unter den Sinterbedingungen zumindest auf die Abmessungen der zu restaurierenden Zahnsituation verringert. Während des Sinterns verringert sich die Porosität innerhalb der Schicht abgeschiedener metallischer und gegebenenfalls keramischer Partikel, und da sich das Volumen des Modells unter den Sinterbedingungen zumindest auf die Abmessungen der zu restaurierenden Zahnsituation verringert, schrumpft auch die abgeschiedene Schicht der Partikel, wobei ihre Innenabmessungen sich den Außenabmessungen der zu restaurierenden Dentalsituation annähern.

Auch gemäß einer vierten alternativen Verfahrensgestaltung besitzt das im erfindungsgemäßen Verfahren einzusetzende Modell größere Abmessungen als die zu restaurierende Zahnsituation. Das Sintern der abgeschiedenen Schicht der metallischen und gegebenenfalls keramischen Partikel erfolgt jedoch nicht auf dem Modell. Dieses wird vielmehr vor dem eigentlichen Sintervorgang durch eine geeignete Behandlung entfernt, vergleiche hierzu die Anmerkungen zur zweiten alternativen Verfahrensgestaltung. Beispielsweise kann ein Wachsmodell durch eine kurze Temperaturbehandlung bei einer Temperatur, die niedriger als die spätere Sintertemperatur, aus der abgeschiedenen Schicht metallischer und eventuell keramischer Partikel herausgeschmolzen bzw. herausgebrannt werden. Voraussetzung für die Durchführbarkeit der vierten alternativen Verfahrensgestaltung ist natürlich, dass die Grünfestigkeit der abgeschiedenen Schicht von Partikeln hoch genug ist, so dass ein Entfernen des Modellmaterials möglich ist und nicht sogleich zu einem Kollabieren der Struktur der metallischen bzw. metallkeramischen Schicht führt.

Der im erfindungsgemäßen Verfahren eingesetzte Schlicker elektrophoretisch abscheidbarer metallischer und gegebenenfalls keramischer Partikel umfasst ein Pulver mit monomodaler, bimodaler oder höhermodaler Partikelgrößenverteilung. Daneben umfasst der Schlicker ein Dispersionsmittel und gegebenenfalls einen Stabilisator.

In einer Pulvermischung, die metallische und zusätzliche keramische Partikel umfasst, führt das Keramikpulver z.B. zu einer vergleichsweise hohen Härte des fertigen Bauteils, z. B. der fertigen Dentalrestauration.

Vorzugsweise hat das Pulver metallischer und eventuell keramischer Partikel eine bimodale oder höhermodale Partikelgrößenverteilung.

Bevorzugt sind Metallpulver, für deren Partikelgrößenverteilung der relativen Möglichkeiten gilt: 0,1 µm < d₅₀ < 20 µm, vorzugsweise 0,1µm < d₅₀ < 10 µm.

Sofern der Schlicker elektrophoretisch abscheidbarer Partikel ein Pulver mit bimodaler Partikelgrößenverteilung umfasst (was bevorzugt ist), stehen die Partikelgrößen an den Orten der beiden Maxima der relativen Häufigkeiten der Partikelgrößenverteilung vorzugsweise zueinander in einem Partikelgrößenverhältnis von größer als 5:1. Die Partikel werden beispielsweise mittels Laserbeugung vermessen.

In einer bevorzugten Ausführung eines solchen Schlickers umfasst der Schlicker ein Pulver mit einem Anteil metallischer und einem Anteil keramischer Partikel, wobei die kleineren Partikel, d.h. das Maximum in der Partikelgrößenverteilungskurve, welches der geringeren Partikelgröße zugeordnet ist, zu dem keramischen Anteil gehören.

Die Verwendung derartiger Schlicker bzw. Pulver führt zu einer elektrophoretisch abgeschiedenen Schicht mit besonders hoher Gründichte (das heißt mit besonders niedriger Porosität).

Die elektrophoretisch abscheidbaren metallischen Partikel können wahlweise aus edelmetallhaltigen oder edelmetallfreien Legierungen bestehen. Eingesetzt werden beispielsweise Edelmetalle und sonstige Metalle wie Gold, Titan, Eisen, Platin, Palladium, Silber, Kobalt, Chrom etc. sowie deren Legierungen. Der Einsatz von goldhaltigen Legierungen ist oftmals bevorzugt.

Elektrophoretisch abscheidbare keramische Partikel sind z. B. Alumina, Zirconia, Spinell, usw.

Bei der Herstellung des Schlickers elektrophoretisch abscheidbarer Partikel beachtet der Fachmann, dass vorzugsweise während der elektrophoretischen Abscheidung keine bzw. nur eine geringe Veränderung der Schlickerzusammensetzung erfolgt. Er berücksichtigt, dass die Sedimentation der Partikel und damit die Entmischung des Schlickers durch die Partikelgröße, den Dichteunterschied zwischen Dispersionsmittel (Flüssigkeit) und Partikel sowie durch die Viskosität des Schlickers beeinflusst wird. Er beachtet, dass kleine Partikel innerhalb des Schlickers langsamer absinken als große und wählt insbesondere deshalb die oben als bevorzugt charakterisierten Partikelgrößenverteilungen. Der Fachmann weiß, dass eine hohe Dichte des Dispersionsmittels (der Flüssigkeit) zu einer hohen Auftriebskraft auf die Partikel und damit zu geringeren Absinkgeschwindigkeiten führt. Zudem ist dem Fachmann bekannt, dass eine hohe Viskosität die Absinkgeschwindigkeit verringert. Der Fachmann wird die Viskosität des Schlickers einstellen, indem er den Feststoffanteil und/oder die Ausgangsviskosität des Dispersionsmittels geeignet wählt. In bevorzugt zu verwendendenden Schlickern liegt der Feststoffanteil des Schlickers in einem Bereich von 20 bis 75 Vol.-%, vorzugsweise im Bereich von 30 bis 60 Vol.-%, bezogen auf das Gesamtvolumen des Schlickers.

Insbesondere um einen hohen Füllgrad des Schlickers zu erhalten, wird bevorzugt mit der bereits weiter oben als bevorzugt angegebenen bimodalen Pulververteilung gearbeitet, vorzugsweise mit Pulvern, die metallische und keramische Partikel umfassen.

Die eingesetzten Pulver können einphasig (bei Abwesenheit keramischer Partikel), zweiphasig oder mehrphasig sein; in manchen Fällen ist der Einsatz zwei- oder mehrphasiger Pulver auch dann vorteilhaft, wenn keine keramischen Partikel eingesetzt werden. Durch Einsatz zwei- oder mehrphasiger Pulver können Komposits erzeugt werden, d.h. Bauteile, die aufgrund des Einsatzes verschiedener Materialien vorteilhafte Eigenschaften der Materialien vereinigen. So lässt sich beispielsweise die Härte und Zähigkeit eines Bauteils gezielt durch den Einsatz verschiedener Materialien beeinflussen. Darüber hinaus kann sich natürlich auch die Schmelztemperatur der Materialien unterscheiden. So lässt sich ein höher schmelz- bzw. sinterbares metallisches oder keramisches Material mit einem niedriger schmelz- bzw. sinterbaren Material kombinieren und damit die benötigte Sintertemperatur senken.

Als vorteilhaft erwiesen hat es sich, einen Schlicker elektrophoretisch abscheidbarer Partikel einzusetzen, der ein Pulver mit bimodaler oder höhermodaler Partikelgrößenverteilung umfasst, wobei die Pulverfraktion mit der größten Partikelgröße kugelförmige Teilchen umfasst oder aus kugelförmigen Teilchen besteht.

In einem erfindungsgemäßen Verfahren wird das Sintern bzw. Ansintern der abgeschiedenen Schicht der Partikel zur Fertigkeitssteigerung vorzugsweise bei einer Temperatur durchgeführt, die im Bereich des 0,45 - bis 0,9-fachen der Schmelztemperatur bzw. Solidustemperatur der am niedrigst schmelzenden bzw. der einzigen metallischen Phase der Schicht liegt. Die Schmelz- bzw. Solidustemperatur wird dabei in Kelvin (K) angegeben.

Das Sintern bzw. Ansintern der abgeschiedenen Schicht der Partikel führt zu einer Verfestigung. Die gewählte Sintertemperatur wird vom verwendeten Material und dem angestrebten Ergebnis des Sintervorganges abhängen. Übliche Sintertemperaturen liegen im Bereich von 600 - 1400°C.

Soll neben einer Verfestigung auch die Porosität verringert und damit die Geometrie der Schicht abgeschiedener Partikel verändert werden, so wird bei Einsatz einphasiger Pulver vorzugsweise eine Sintertemperatur im Bereich des 0,6- bis 0,9-fachen der Schmelztemperatur bzw. der Solidustemperatur gewählt. Bei mehrphasigen Pulvern (Pulvermischungen) wird in diesem Fall bevorzugt eine Sintertemperatur gewählt, die im Bereich des 0,6- bis 0,9-fachen der Schmelztemperatur bzw. Solidustemperatur der am niedrigst schmelzenden metallischen Phase der Schicht (des Pulvers) liegt. Bei Verwendung mehrphasiger Pulver kann jedoch auch oberhalb der Schmelztemperatur bzw. Solidustemperatur der am niedrigst schmelzenden Phase gesintert werden (Schmelzsintern).

Über die Sintertemperatur kann der Fachmann das Gefüge der resultierenden gesinterten metallischen Schicht einstellen. Bei hohen Sintertemperaturen wird das Kornwachstum beschleunigt, so dass ein vergleichsweise grobes Gefüge entsteht; gleichzeitig ist die benötigte Sinterzeit recht kurz. Bei niedrigeren Sintertemperaturen verhält es sich gerade umgekehrt; die benötigte Sinterzeit ist recht hoch, es entsteht jedoch ein feineres Gefüge.

Soll die Wärmebehandlung lediglich zur Festigkeitssteigerung dienen, ohne dass damit eine Längenänderung von 0,1% oder mehr verbunden sein soll, wird die Wärmebehandlung (Ansintern) der abgeschiedenen Schicht vorzugsweise bei einer Temperatur durchgeführt, die im Bereich des 0,45- bis 0,6-fachen der Schmelztemperatur bzw. Solidustemperatur der am niedrigst schmelzenden bzw. der einzigen metallischen Phase der Schicht liegt. Bei derart niedrigen Temperaturen kommt es zwar zu einer Festigkeitssteigerung, da benachbarte metallische Partikel über Sinterstege miteinander verbunden werden, die Geometrie des Grünkörpers wird jedoch bewahrt. Es resultiert ein angesinterter Grünkörper mit verbliebener Porosität.

Die verbliebene Porosität angesinterter oder gesinterter Schichten abgeschiedener Partikel kann anschließend mit einem niedrig schmelzenden Material aufgefüllt werden, vorzugsweise unter Verwendung eines Metalls, z. B. einer niedrig schmelzenden Legierung, oder unter Verwendung eines vorzugsweise niedrig schmelzenden Glases oder eines vorzugsweise niedrig schmelzenden Harzes oder Kunststoffes. Auf diese Weise lassen sich Komposits erzeugen, die in manchen Fällen gewünscht sind. "Niedrig schmelzend" sind dabei Materialien, deren Schmelzpunkt nicht höher ist als das 0,8-fache der Schmelztemperatur bzw. der Solidustemperatur der am niedrigst schmelzenden oder einzigen metallischen Phase der abgeschiedenen Schicht.

Das Sintern bzw. das Ansintern der abgeschiedenen Schicht der metallischen sowie gegebenenfalls keramischen Partikel kann unter normaler Atmosphäre erfolgen, sofern Edelmetalle bzw. Edelmetalllegierungen eingesetzt wurden. Bei Verwendung unedler Metalle bzw. Legierungen wird der Fachmann die Sinteratmosphäre gezielt wählen. Hierbei wird er berücksichtigen, dass Sauerstoff, Stickstoff und Kohlenstoff mit unedlen Metallen bzw. Legierungsbestandteilen reagieren können. Insbesondere kann es zur Oxidation der Partikeloberflächen kommen, was zu einem Formkörper mit verschlechterten mechanischen Eigenschaften führt. Es hat sich als vorteilhaft erwiesen, das Sintern der abgeschiedenen Schicht der Partikel mit dem Ziel Verringerung der Porosität bzw. das Ansintern der abgeschiedenen Schicht der Partikel zur bloßen Erhöhung der Festigkeit in sauerstoffarmer Atmosphäre durchzuführen (der Anteil an Sauerstoff liegt unter dem Anteil an Sauerstoff in der Luft). Abhängig von der Affinität des eingesetzten Metalls bzw. der eingesetzten Legierungselemente wird vorzugsweise auch der Stickstoff- und/oder Kohlenstoffanteil in der Sinteratmosphäre reduziert. In machen Fällen ist es vorteilhaft, die zum Sintern bzw. Ansintern eingesetzte Ofenkammer mit Argon zu spülen; insbesondere dann lässt sich eine oxidgasfreie bzw. oxidgasarme Ofenkammeratmosphäre (Inertgasatmosphäre) erreichen.

In anderen Fällen wird der Fachmann jedoch gezielt Oxidationsgase zugeben, um die Bildung eines Komposits zu erreichen.

Besonders bevorzugt ist eine Ausgestaltung des erfindungsgemäßen Verfahrens, in der der Anteil metallischer Partikel in dem eingesetzten Schlicker 75-100 Gew.-% beträgt, vorzugsweise 100 Gew.-%, bezogen auf die Gesamtmasse an Partikeln im Schlicker, wobei
(i) die metallischen Partikel aus edelmetallhaltigen Legierungen oder Edelmetallen wie Gold, Platin, Palladium oder Silber bestehen
   und/oder
(ii) das Sintern der abgeschiedenen Schicht der metallischen Partikel bzw. das Ansintern der abgeschiedenen Schicht der metallischen Partikel in sauerstoffarmer Atmosphäre durchgeführt wird (dies ist insbesondere wichtig bei Einsatz von edelmetallfreien Legierungen).Als Dispersionsmittel zur Verwendung in einem erfindungsgemäß einzusetzenden Schlicker werden vorzugsweise Dispersionsmittel aus der Gruppe ausgewählt, die aus Wasser, Alkoholen und deren Mischungen besteht. Alternativ kann man auch organische Säuren und deren Ester, Alkoholderivate, organische Stickstoffverbindungen (Amide, Nitroverbindungen, Nitrile), Aldehyde und Ketone, organische Schwefelverbindungen (Sulfoxide, etc) und Ether verwenden. Ein Dispersionsmittel sollte, da die Abscheidegeschwindigkeit der Partikel unter anderem abhängig ist von der Dielektrizitätskostante des Dispersionsmittels, eine Dielektrizitätszahl von zumindest 10 besitzen (zum Vergleich: Wasser (~80), n-Propanol (~20)) und vorzugsweise gut handhabbar sein (keine Kanzerogenität, keine Toxizität).

Besonders bevorzugt ist der Einsatz von Wasser, Ethanol, Isopropanol, n-Propanol und deren Mischungen.

Sofern in einem erfindungsgemäß einzusetzenden Schlicker ein Stabilisator vorliegt, ist dieser vorzugsweise ausgewählt aus der Gruppe bestehend aus: elektrostatisch stabilisierenden Stabilisatoren, elektrosterisch stabilisierenden Stabilisatoren und deren Mischungen.

Ziel des Einsatzes von Stabilisatoren ist die Herstellung einer stabilen Dispersion, bei der die Partikel eine Ladung aufweisen, so dass eine gleichmäßige elektrophoretische Abscheidung möglich ist. Ursache für die Partikelbewegung innerhalb des Schlickers ist dabei die Wechselwirkung zwischen dem äußeren elektrischen Feld und der Ladung des Partikels.

Das Versehen der Partikeloberflächen mit elektrischen Ladungen kann 1) durch chemische Reaktionen an der Partikeloberfläche und 2) durch spezifische Adsorption/Desorption von Ionen erfolgen. Bei der elektrostatischen Stabilisierung wird der Tendenz der dispergierten metallischen Partikel, sich auf Grund von Van-der-Waals-Kräften (Dipol bindungen) zu koagulieren, durch elektrostatische Abstoßung entgegengewirkt. Bei der elektrosterischen Stabilisierung wird ebenfalls von dem Effekt der elektrostatischen Abstoßung Gebrauch gemacht, hinzu kommt jedoch eine sterische Hinderung, welche einer Koagulation entgegensteht.

Vorzugsweise werden die elektrostatisch bzw. elektrosterisch stabilisierenden Stabilisatoren ausgewählt aus der Gruppe bestehend aus: organische und anorganische Basen und Säuren, deren Salze sowie deren Kationen bzw.

Anionen, Komplexbildner, Polymere (insbesondere als Ionen oder in flüssiger Form) und deren Mischungen.

### Beispiele für geeignete Stabilisatoren sind:

Essigsäure (organische Säure), 4-Hydrxybenzoesäure (organische Säure), Ethylendiamintetraacetat (dissoziierter Rest eines Salzes einer organische Säure, der einen Komplex bildet), Harnstoff (organische Verbindung einer anorganischen Base), Triethanolamin (organische Verbindung einer anorganischen Base), Polyethyleneimin (Polymer).

In dem erfindungsgemäßen Verfahren ist es in machen Fällen sinnvoll, dem Schlicker ein Bindemittel zu zusetzen, das dann gemeinsam mit den metallischen Partikeln abgeschieden wird. Zusätzlich oder alternativ kann auf die abgeschiedene Schicht der Partikel vor der thermischen (Sintern, Ansintern, Wärmebehandlung zum Entfernen des Modells) oder mechanischen Behandlung ein Bindemittel aufgetragen werden. Das Bindemittel trägt bei einer derartigen Verfahrensgestaltung zu einer Erhöhung der Grünkörperfestigkeit und -härte bei. Als Bindemittel können insbesondere Silikonharze, Silane, Siloxane, Silikonester, Acrylate, Polyvinylalkohole oder sonstige Polymere eingesetzt werden, z. B. üblicherweise in Farben, Lacken und der Baustoffindustrie eingesetzte Bindemittel. Die Bindemittel sind anorganischer oder organischer Natur; organische Bindemittel sind bevorzugt, da sie nicht im gesinterten Gerüst verbleiben. Der Einsatz eines Bindemittels führt zu einer Verringerung der Gefahr, dass der Grünkörper beim Handling, der Nachbearbeitung oder beim Trocknen zerstört bzw. beschädigt wird. Nach der elektrophoretischen Beschichtung eines Arbeitsmodells kann die resultierende dentale Restauration beispielsweise mit einem Skalpell oder einem Handfräser nachbearbeitet werden. So wird beispielsweise die Präparationsgrenze freigelegt oder die Schichtstärke an gewünschten Stellen verringert. Hierbei ist ein stabiler Grünkörper vorteilhaft. Für die erfindungsgemäße Verfahrensvariante, bei der das Gerüst vor dem Sintern bzw. Ansintern vom Modell getrennt wird, ist ebenfalls eine erhöhte Grünfestigkeit erforderlich. In diesem Fall erfolgt nämlich eine thermische, mechanische oder chemische Behandlung des Modells und des Grünkörpers. Anschließend entfällt der Halt, den der Grünkörper durch das Arbeitsmodell erhalten hat. Nach der Entnahme des beschichteten Negativmodells aus dem Schlicker beginnt die abgeschiedene Schicht metallischer und gegebenenfalls keramischer Partikel zu trocknen. In Abhängigkeit von der Partikelgrößenverteilung kann es zu Trockenschwindungen kommen (bei kleinen Partikeln stärker ausgeprägt als bei großen). Damit dies nicht zu Trocknungsrissen führt, wird die Festigkeit der Schicht vorzugsweise durch Bindemittel heraufgesetzt.

Die Erfindung betrifft auch ein Kit zur Durchführung eines erfindungsgemäßen Verfahrens, umfassend:
- Metallpulver, Dispersionsmittel und gegebenenfalls Stabilisator und/oder Bindemittel und/oder Keramikpulver zur Herstellung eines Schlickers elektrophoretisch abscheidbarer metallischer Partikel;
- Elektrophoreseeinrichtung zur elektrophoretischen Abscheidung metallischer Partikel.

Hinsichtlich bevorzugter Ausgestaltungen gilt das oben zum erf.- gem. Verfahren Gesagte entsprechend.

Die Erfindung betrifft ferner die Verwendung eines Metallpulvers oder eines Schlickers umfassend elektrophoretisch abscheidbare metallische Partikel (sowie gegebenenfalls keramische Partikel) zur elektrophoretischen Abscheidung metallischer (und gegebenenfalls keramischer) Partikel auf der Oberfläche eines Negativmodells, z. B. des Modells einer zu restaurierenden Zahnsituation. Besonders bevorzugt ist hierbei wieder die Verwendung eines Metallpulvers in Abwesenheit eines Keramikpulvers bzw. eines Schlickers , der frei ist von Keramikpartikeln, vergleiche hierzu die Ausführungen zu bevorzugten erfindungsgemäßen Verfahren.

Hinsichtlich bevorzugter Ausgestaltungen gilt jeweils das oben gesagte entsprechend.

Weitere Aspekte und bevorzugte Ausgestaltungen der Erfindung ergeben sich aus den beigefügten Patentansprüchen.

Nachfolgend wird die Erfindung anhand von Ausführungsbeispielen näher erläutert:

### Beispiel 1:

### Herstellung einer sintermetallischen dentalen Schicht

Ein Duplikatstumpf wird aus temperaturbeständigem Abformaterial (phosphatgebundende Einbettmasse) hergestellt und dient als Modell einer zu restaurierenden Zahnsituation. Auf die Oberfläche des Duplikatstumpfes wird dünn Silberleitlack aufgetragen und auf diese Weise die Oberfläche des Modells elektrisch leitfähig gemacht. Die leitfähig gemachte Oberfläche des Duplikatstumpfes wird elektrisch kontaktiert und der Duplikatstumpf in einen Schlicker elektrophoretisch abscheidbarer metallischer Partikel gehängt. Der Schlicker umfasst 210 g Pulver einer edelmetallfreien Dentallegierung, 20 g Ethanol als Dispersionsmittel sowie 4,2 g Harnstoff (Stabilisator). Das Pulver besitzt eine maximale Partikelgröße von 10 µm, eine monomodale Verteilung und einen mittleren Partikeldurchmesser von d50=5µm. Die Dentallegierung ist eine Cobaltchromlegierung, die als weitere Bestandteile Molybdän, Wolfram, Silizium und Eisen enthält. Die eingesetzte Menge an Harnstoff entspricht 2 Gew.-%, bezogen auf die Masse des Pulvers der Dentallegierung. In dem Schlicker befindet sich zudem eine Gegenelektrode. Der kleinste Elektrodenabstand der Elektroden beträgt 15 mm. Durch Anlegen einer Spannung von 40 V zwischen der elektrisch leitfähigen Oberfläche des Duplikatstumpfes und der Gegenelektrode bildet sich innerhalb von 45 Sekunden eine 0,5mm dicke Schicht metallischer Partikel auf der mit Silberleitlack versehenen Oberfläche des Duplikatstumpfes. Der Duplikatstumpf wird zusammen mit der abgeschiedenen Schicht der metallischen Partikel in einem Ofen thermisch behandelt. Dabei wird eine Sintertemperatur von 900 °C eingestellt. Der Ofen wird während dieser thermischen Behandlung mit Argon gespült. Die Solidustemperatur der eingesetzten Dentallegierung liegt bei 1270 °C (Liquidustempera tur: 1380 °C). Das Verhältnis von Sintertemperatur zu Solidustemperatur ist demnach 0,76. Während des Sintervorganges verringert sich die Porosität der metallischen Schicht. Da es sich bei dem Duplikatstumpf um ein Modell aus temperaturbeständigem Abformaterial handelt, unterliegt dieses keinem Sinterschrumpf, die Außengeometrie (die Außenabmessungen) des Stumpfes bleibt erhalten. Die metallische Schicht bewahrt daher ihre Innengeometrie, es kommt lediglich zu einer Reduzierung der Schichtdicke. Aus der gesinterten metallischen Schicht lässt sich der Duplikatstumpf durch Herausstrahlen oder Ätzen entfernen. Die sintermetallische Schicht kann als dentale Krone eingesetzt werden.

### Beispiel 2:

### Herstellung eines Zahnrades

Beispiel 2 betrifft die Herstellung eines Zahnrades in Form eines Stirnrades. Der Arbeitsdurchmesser (Teilkreisdurchmesser) dieses Stirnrades liegt bei 48 mm, die Zähnezahl bei z= 24. Hieraus ergibt sich ein Modul von m=2 mm. Das Zahnrad hat eine Innenbohrung von 10 mm.

Die Erzeugung des Datenmodells für einen späteren Rapid-Prototyping-Prozess zur Herstellung des Zahnrads erfolgte am Rechner. Dabei wurde berücksichtigt, dass der anhand des Datenmodells erzeugte metallische Grünkörper beim Sintern schrumpft. Es wurde deshalb eine Geometrievergrößerung des Datenmodells gemäß dieser Volumenabnahme vorgenommen. Anhand der CAD-Daten (Datenmodell) des metallischen Grünkörpers des Zahnrades wurde am Rechner ein Daten-Negativ des um den Sinterschrumpf vergrößerten Zahnrades erstellt, das so ausgerichtet war, dass dessen Achse senkrecht zur Horizontalen steht. Zudem war dieses Daten-Negativ in horizontale Lagen gleicher Dicke unterteilt. Das echte Negativ wurde dann mittels des Rapid-Prototyping-Verfahrens der Stereolithographie hergestellt. Zum Herstellen wurde ein lichtaushärtendes Harz verwendet, das durch die Zugabe von Ruß elektrisch leitend gemacht wurde. Auf ein elektrisch nichtleitendes Substrat wurde stereolithographisch eine erste Lage des Gemischs aus lichtaushärtendem Harz und Ruß aufgetragen und vollständig ausgehärtet. Anschließend erfolgte die weitere Auftragung lagenweise mit anschließender, selektiver Belichtung gemäß dem erzeugten Datensatz.

Nach dem Aufbau und dem selektiven Aushärten der letzten Schicht, wurde das Substrat zusammen mit dem Rapid-Prototyping-Modell dem Stereolithographiegerät entnommen. Anschließend wurde das Rapid-Prototyping-Modell von dem überschüssigen, noch flüssigen Harz befreit. Zurück blieb das Rapid-Prototyping-Modell in Form einer elektrisch leitenden Negativform des um den antizipierten Sinterschrumpf vergrößerten Zahnrades auf dem nichtleitenden Substrat. Diese Negativ-Form wurde elektrisch kontaktiert und in einen metallischen Schlicker getaucht. Die Zusammensetzung des Schlickers entspricht der aus Beispiel 1.

Nach Anlegen einer Spannung wurde auf der elektrisch leitfähigen Oberfläche des Rapid-Prototyping-Modells der Schlicker elektrophoretisch als Metallschicht abgeschieden. Nachdem das Rapid-Prototyping-Modell (Negativ) mit der Metallschicht ausgefüllt war, wurde das Rapid-Prototyping-Modell aus dem Schlickerbad entnommen. Das Rapid-Prototyping-Modell wurde zusammen mit dem Substrat, mit dem das Rapid-Prototyping-Modell verbunden ist, in eine Fräse eingespannt. Anschließend erfolgt das Abtragen überschüssigen abgeschiedenen Schlickers von der nach oben weisenden Oberfläche des Rapid-Prototyping-Modells durch Fräsen. In diesem Fräs-Prozess wurde auch das Durchgangsloch des späteren Zahnrades erzeugt und eine gute Planparallelität der Zahnradseiten hergestellt. In einer anschließenden thermischen Behandlung (siehe oben) erfolgte das Entfernen des Rapid-Prototyping-Modells sowie das Dichtsintern des metallischen Bauteils in Form des Zahnrades.

### Beispiel 3:

### Herstellung eines Zahnrades

In Beispiel 3 wurde die Negativform (als Negativmodell) eines Zahnrads auf die in Beispiel 2 beschriebene Weise, aber in der Größe des benötigten Bauteils erzeugt. Nach der Erzeugung eines metallischen Grünkörpers auf die in Beispiel 2 beschriebene Weise wurde dessen metallisches Gefüge nur angesintert. Zur Steigerung der Festigkeit und der Oberflächenqualität erfolgt die Infiltration des Bauteils mit Epoxydharz. Die Infiltration mit dem Epoxydharz verläuft in Zyklen und wird aufgrund höherer Viskosität des Harzes unter Vakuum durchgeführt. Trotzdem wird das Metallsintermaterial nur in den Randbereichen vollständig infiltriert. Es verbleibt eine Restporösität von ca. 15 % im Inneren des Bauteils. Zum anschließenden Aushärten des Harzes ist lediglich eine Temperatur von 160° C notwendig, so dass keinerlei Veränderung der Geometrie stattfindet und die hohe Genauigkeit des Bauprozesses bestehen bleibt.

### Anmerkung zu den Beispielen 2 und 3:

Auf die beschriebene oder analoge Weise lassen sich auch andere Bauteile herstellen, wie beispielsweise Spritzgussformen, Gesenke, Spiegel, Goldgerüste für die Dentaltechnik, Druckplatten zur Aufbringen einer Maserung bei der Herstellung von Kunstleder und Keramiksonderteilen. Besonders vorteilhaft können erfindungsgemäße Verfahren zur Herstellung von Kleinstserien von Bauteilen komplexer Geometrie eingesetzt werden.

## Patentansprüche

**1.** Verfahren zur Herstellung eines metallischen Bauteils, mit folgenden Schritten:
- Bereitstellen eines Negativmodells für das Bauteil, welches (i) zumindest an seiner Oberfläche elektrisch leitfähig ist oder leitfähig gemacht ist oder (ii) porös ist und im Innern eine Elektrode umfasst,
- Bereitstellen eines Schlickers, der elektrophoretisch abscheidbare metallische und gegebenenfalls zusätzlich keramische Partikel umfasst,
- Elektrophoretisches Abscheiden einer Schicht der Partikel aus dem Schlicker auf der Oberfläche des Modells,
- Sintern oder Ansintern der abgeschiedenen Schicht der Partikel.

**2.** Verfahren zur Herstellung einer Dentalrestauration auf Metallbasis, mit folgenden Schritten:
- Bereitstellen eines Modells der zu restaurierenden Zahnsituation, welches (i) zumindest an seiner Oberfläche elektrisch leitfähig ist oder leitfähig gemacht ist oder (ii) porös ist und im Innern eine Elektrode umfasst,
- Bereitstellen eines Schlickers, der elektrophoretisch abscheidbare metallische und gegebenenfalls zusätzlich keramische Partikel umfasst,
- Elektrophoretisches Abscheiden einer Schicht der Partikel aus dem Schlicker auf der Oberfläche des Modells,
- Sintern oder Ansintern der abgeschiedenen Schicht der Partikel.

**3.** Verfahren nach Anspruch 1 oder 2, wobei der Anteil metallischer Partikel in dem Schlicker 50-100 Gew.-%, bevorzugt 75-100 Gew.-%, beträgt, bezogen auf die Gesamtmasse metallischer und keramischer Partikel.

**4.** Verfahren nach einem der Ansprüche 1 bis 3, wobei der Anteil metallischer Partikel in dem Schlicker 100 Gew.-% beträgt, bezogen auf die Gesamtmasse an Partikeln.

**5.** Verfahren nach einem der vorangehenden Ansprüche, wobei das Negativmodell bzw. das zumindest an seiner Oberfläche elektrisch leitfähige oder leitfähig gemachte Modell der zu restaurierenden Zahnsituation umfasst:
- ein nicht nur an seiner Oberfläche elektrisch leitfähiges Modellmaterial ohne zusätzliche oberflächliche Beschichtung
oder
- ein elektrisch nicht leitendes Modellmaterial mit einer oberflächlich darauf angeordneten Beschichtung aus einer leitfähigen Substanz.

**6.** Verfahren nach einem der vorangehenden Ansprüche, wobei die Porengröße des porösen Modells so an die Partikelgröße der metallischen und gegebenenfalls keramischen Partikel angepasst ist, dass die Partikel bei der Elektrophorese nicht bis zur Elektrode im Innern des Modells vordringen können.

**7.** Verfahren nach einem der vorangehenden Ansprüche 2 bis 6, wobei das Modell die Abmessungen der zu restaurierenden Zahnsituation besitzt, das Sintern der abgeschiedenen Schicht der Partikel auf dem Modell erfolgt und das Modellmaterial so gewählt ist, dass das Modell unter den Sinterbedingungen eine relative Längenänderung von nicht mehr als 0,2% erfährt.

**8.** Verfahren nach Anspruch 7, wobei das Modell aus der gesinterten Schicht der Partikel mechanisch oder chemisch entfernt wird.

**9.** Verfahren nach einem der vorangehenden Ansprüche 2 bis 6, wobei das Modell die Abmessungen der zu restaurierenden Zahnsituation besitzt, die abgeschiedene Schicht der beteiligten Partikel angesintert und gegebenenfalls zuvor einer Wärmebehandlung unterzogen wird, wobei beim Ansintern bzw. der vorausgehenden Wärmebehandlung das Modellmaterial zusammenschrumpft bzw. aus der metallischen Schicht herausbrennt.

**10.** Verfahren nach einem der Ansprüche 2 bis 6, wobei das Modell größere Abmessungen besitzt als die zu restaurierende Zahnsituation, das Sintern der abgeschiedenen Schicht der Partikel auf dem Modell erfolgt und das Modellmaterial so gewählt ist, dass sich das Volumen des Modells unter den Sinterbedingungen zumindest auf die Abmessungen der zu restaurierenden Zahnsituation verringert.

**11.** Verfahren nach einem der vorangehenden Ansprüche, wobei der Schlicker elektrophoretisch abscheidbarer Partikel ein dispergiertes Pulver mit monomodaler, bimodaler oder höhermodaler Partikelgrößenverteilung, ein Dispersionsmittel und gegebenenfalls einen Stabilisator umfasst.

**12.** Verfahren nach Anspruch 11, wobei für die Partikelgrößenverteilung des Pulvers gilt: 0,1 µm < d₅₀ < 20 µm.

**13.** Verfahren nach Anspruch 11 oder 12, wobei der Schlicker elektrophoretisch abscheidbarer Partikel ein Pulver mit bimodaler Partikelgrößenverteilung umfasst, wobei die Partikelgrößen an den Orten der beiden Maxima der relativen Häufigkeiten der Partikelgrößenverteilung zueinander in einem Partikelgrößenverhältnis von größer als 5:1 stehen.

**14.** Verfahren nach einem der vorangehenden Ansprüche, wobei die metallischen Partikel aus edelmetallhaltigen Legierungen, Gold oder Platin bestehen. 15. Verfahren nach einem der vorangehenden Ansprüche, wobei das Sintern der abgeschiedenen Schicht der Partikel bzw. das Ansintern der abgeschiedenen Schicht der Partikel in sauerstoffarmer Atmosphäre durchgeführt wird.

**16.** Verfahren nach einem der vorangehenden Ansprüche 4 bis 15, wobei
(i) die metallischen Partikel aus edelmetallhaltigen Legierungen oder Edelmetallen wie Gold, Platin, Palladium oder Silber bestehen
und/oder
(ii) das Sintern der abgeschiedenen Schicht der metallischen Partikel bzw. das Ansintern der abgeschiedenen Schicht der metallischen Partikel in sauerstoffarmer Atmosphäre durchgeführt wird.

**17.** Verfahren nach einem der vorangehenden Ansprüche, wobei der Feststoffanteil des Schlickers in einem Bereich von 20 bis 75 Vol.-% liegt, vorzugsweise im Bereich von 30 bis 60 Vol.-%, bezogen auf das Gesamtvolumen des Schlickers.

**18.** Verfahren nach einem der vorangehenden Ansprüche 11 bis 17, wobei der Schlicker elektrophoretisch abscheidbarer Partikel ein Pulver mit bimodaler oder höhermodaler Partikelgrößenverteilung umfasst, dessen Fraktion mit der größten Partikelgröße kugelförmige Teilchen umfasst oder aus kugelförmigen Teilchen besteht.

**19.** Verfahren nach einem der vorangehenden Ansprüche, wobei das Sintern bzw. Ansintern der abgeschiedenen Schicht der Partikel bei einer Temperatur durchgeführt wird, die im Bereich des 0,45- bis 0,9-fachen der Schmelztemperatur bzw. Solidustemperatur (in K) der am niedrigsten schmelzenden bzw. der einzigen metallischen Phase der Schicht liegt.

**20.** Verfahren nach Anspruch 19, wobei das Sintern bzw. Ansintern der abgeschiedenen Schicht der metallischen Partikel
(i) zur Festigkeitssteigerung und Verringerung der Porosität der Schicht um mindestens 5%, bezogen auf die Porosität vor dem Sintern bzw. Ansintern, bei einer Temperatur durchgeführt wird, die im Bereich des 0,6- bis 0,9-fachen der Schmelztemperatur bzw. Solidustemperatur der am niedrigsten schmelzenden bzw. der einzigen metallischen Phase der Schicht liegt oder
(ii) zur Festigkeitssteigerung, bei Verringerung der Porosität der Schicht um weniger als 5%, bezogen auf die Porosität vor dem Sintern bzw. Ansintern, bei einer Temperatur durchgeführt wird, die im Bereich des 0,45- bis 0,6-fachen der Schmelztemperatur bzw. Solidustemperatur der am niedrigsten schmelzenden bzw. der einzigen metallischen Phase der Schicht liegt.

**21.** Verfahren nach einem der vorangehenden Ansprüche, wobei eine nach dem Sintern oder Ansintern verbliebene Porosität mit Glas, Harz, Kunststoff oder Metall infiltriert wird.

**22.** Verfahren nach einem der Ansprüche 11 bis 21, wobei der Stabilisator ausgewählt ist aus der Gruppe bestehend aus: elektrostatisch stabilisierenden Stabilisatoren, elektrosterisch stabilisierenden Stabilisatoren und deren Mischungen.

**23.** Verfahren nach Anspruch 22, wobei der Stabilisator ausgewählt ist aus der Gruppe bestehend aus: organische und anorganische Basen und Säuren, deren Salze sowie deren Kationen bzw. Anionen, Komplexbildner, Polymere und deren Mischungen.

**24.** Verfahren nach einem der vorangehenden Ansprüche, wobei dem Schlicker ein Bindemittel zugesetzt ist, das gemeinsam mit den Partikeln abgeschieden wird und/oder auf die abgeschiedene Schicht der metallischen Partikel vor der thermischen oder mechanischen Behandlung ein Bindemittel aufgetragen wird.

**25.** Bauteil, insbesondere Dentalrestauration, auf Metallbasis herstellbar nach einem Verfahren gemäß einem der vorangehenden Ansprüche.

**26.** Kit zur Durchführung eines Verfahren gemäß einem der Ansprüche 1 bis 25, umfassend:
- Metallpulver, Dispersionsmittel, und gegebenenfalls Stabilisator und/oder Bindemittel und/oder Keramikpulver zur Herstellung eines Schlickers elektrophoretisch abscheidbarer Partikel;
- Elektrophoreseeinrichtung zur elektrophoretischen Abscheidung der Partikel.

**27.** Verwendung eines Metallpulvers oder eines Schlickers umfassend elektrophoretisch abscheidbare metallische Partikel zur elektrophoretischen Abscheidung metallischer Partikel auf der Oberfläche eines Negativmodells.
